# EUROPEAN PATENT APPLICATION

(11) **EP 4 623 901 A1**
(43) Date of publication of application: **01.10.2025**
(21) Application number: 23917288.5
(22) Date of filing: 18.12.2023
(51) Int. Cl.: A61K 9/06, A61K 47/59, A61K 47/58, A61K 31/728, A61K 31/727, A61P 17/00, A61K 8/85, A61K 8/88, A61K 8/84, A61K 8/81, A61K 8/73, A61K 8/04, A61Q 19/00

(54) **EFFICIENT TRANSDERMAL DELIVERY SYSTEM FOR ACIDIC GROUP-CONTAINING BIOMATERIAL**

(30) Priority: 17.01.2023 CN 202310090280
(71) Applicant: Hangzhou Tito Biotechnology Partnership Enterprise (Limited Partnership), Hangzhou, Zhejiang Province 311222 (CN)
(72) Inventor: SHEN, Youqing, Hangzhou, Zhejiang 310058 (CN); SUN, Rui, Hangzhou, Zhejiang 310058 (CN); FENG, Weiwei, Hangzhou, Zhejiang 310058 (CN)
(74) Representative: Elzaburu S.L.P.
(86) International application number: PCT/CN2023/139392
(87) International publication number: WO 2024/152821

(57) **Abstract**

Provided is an efficient transdermal delivery system based on an acidic group-containing biomaterial produced by bonding or physically compounding a tertiary amine oxide group-containing polymer to an acidic group-containing biomaterial or an acidic group-containing biomaterial nanogel. The efficient transdermal delivery system does not require a subcutaneous injection. After being smeared or coated on a skin, the transdermal delivery system can effectively penetrate through the stratum corneum of the skin and enter the subcutaneous layers to exert prominent medical aesthetic effects such as wrinkle and fold correction, or to achieve the transdermal delivery of heparin for thrombolysis, or to achieve the delivery of a drug.

## Description

### TECHNICAL FIELD

The present disclosure relates to the technical field of biomaterials, and in particular to an efficient transdermal delivery system for acidic group-containing biomaterials.

### BACKGROUND

Acidic group-containing biomacromolecules have extensive applications. For example, hyaluronic acid, also known as hyaluronan, was first approved by the Food and Drug Administration (FDA) in 2003 for wrinkle removal. Hyaluronic acid is now widely used as a cosmetic filler in the medical aesthetics industry. Hyaluronic acid is typically injected into the dermis layer to play moisturizing, lubricating, and shaping roles. In the minimally invasive cosmetic procedures, hyaluronic acid can serve as a filler for rhinoplasty, chin enhancement, lip augmentation, and wrinkle reduction, or it can serve as hyaluronic acid mesotherapy. Unmodified natural linear-chain hyaluronic acid is easily degraded by enzymes, has a half-life period of about 1 d to 21 d in the skin, eyes, joints, and other parts, and is easy to diffuse to other parts, which limits the application of hyaluronic acid in the medical aesthetic industry. Hyaluronic acid can be chemically crosslinked to produce a polymer gel with a three-dimensional network structure, which can effectively slow the enzymatic degradation of hyaluronic acid and overcome the shortcomings of hyaluronic acid, such as short half-life periods in tissues, rapid degradation, and easy diffusion. Other acid-group containing biomaterials including chondroitin sulfate, sodium alginate, and polysaccharide sulfate such as heparin and heparan sulfate, which can be used for suppressing thrombosis, reducing blood viscosity, dilating peripheral blood vessels, and inhibiting the generation of thrombi in arteries and veins, thereby effectively improving functions such as microcirculation.

However, the characteristics of these biomacromolecular materials make these biomacromolecular materials fail to permeate the skin and other epithelial tissues. Therefore, hyaluronic acid and nanogels thereof must be subcutaneously injected into a desired part for shaping. Currently, crosslinked hyaluronic acid can only persist for half a year to a year *in vivo* and usually needs to be filled repeatedly every six months or a year to maintain the plastic effect. Injection of crosslinked hyaluronic acid may cause side effects such as arterial embolism (Zhang Lei et al., The Hyaluronidase Enzymolysis Experiment Research of Hyaluronic Acid in The Arteries, Chinese Journal of Plastic Surgery, 2017, 33 (Z1): 115-121), fibrosis, local hematoma and even infection, and anxiety and foreign body sensation of patients. Acidic group-containing biomaterials, such as heparin, must be injected intravenously for thrombolytic therapy.

### SUMMARY

An objective of the present disclosure is to provide an efficient transdermal delivery system for acidic group-containing biomaterials. The transdermal delivery system does not require a subcutaneous injection. After being smeared or coated on a skin, the transdermal delivery system can effectively penetrate through the stratum corneum of the skin and enter the subcutaneous layers to exert prominent medical aesthetic effects such as wrinkle and fold correction, or to achieve the transdermal delivery of heparin for thrombolysis, or to achieve the delivery of a drug.

To solve the technical problems, the present disclosure adopts a first technical solution as follows:
The present disclosure provides an efficient transdermal delivery system for acidic group-containing biomaterials produced by bonding a tertiary amine oxide group-containing polymer (PNO) to an acidic group-containing biomaterial or an acidic group-containing biomaterial nanogel, where the tertiary amine oxide group-containing polymer (PNO) has a structural formula shown in any one of formulas I to III: 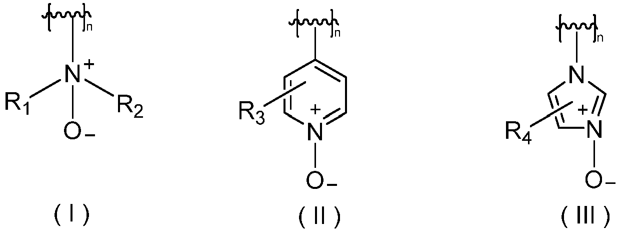 where
R₁ and R₂ each are selected from C₁-C₆ alkyl, substituted alkyl, aryl, or substituted aryl; and
R₃ and R₄ each are selected from hydrogen, halogen, cyano, alkoxy, C₁-C₆ alkyl, substituted alkyl, aryl, or substituted aryl.

A substituent of the substituted alkyl can be one or more of halogen, hydroxyl, alkoxy, amino, and cyano. A substituent of the substituted aryl can be one or more of halogen, hydroxyl, alkoxy, amino, and cyano. Alkyl of the substituted alkyl refers to C₁-C₆ alkyl.

To solve the technical problems, the present disclosure adopts a second technical solution as follows: The present disclosure provides an efficient transdermal delivery system for an acidic group-containing biomaterial, which is a complex produced by mixing an acidic group-containing biomaterial or an acidic group-containing biomaterial nanogel with a tertiary amine oxide group-containing polymer (PNO), where the tertiary amine oxide group-containing polymer (PNO) has a structural formula shown in any one of formulas I to III: 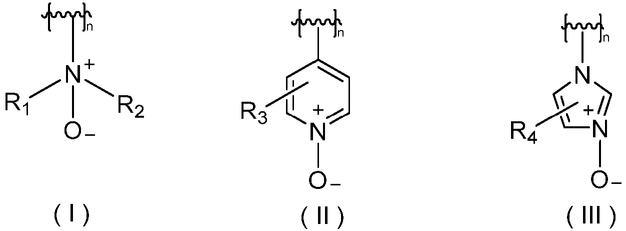 where
R₁ and R₂ each are selected from C₁-C₆ alkyl, substituted alkyl, aryl, or substituted aryl; and
R₃ and R₄ each are selected from hydrogen, halogen, alkoxy, cyano, C₁-C₆ alkyl, substituted alkyl, aryl, or substituted aryl.

A substituent of the substituted alkyl can be one or more of halogen, hydroxyl, alkoxy, amino, and cyano. A substituent of the substituted aryl can be one or more of halogen, hydroxyl, alkoxy, amino, and cyano. Alkyl of the substituted alkyl refers to C₁-C₆ alkyl.

The inventors have found that an oxygen anion of PNO can be protonated by an acid group of an acid group-containing compound. Thus, PNO can bind an acidic group-containing molecule such as hyaluronic acid, heparin, or a crosslinked nanogel thereof through an electrostatic interaction to produce a complex.

The inventors have found through further research that PNO has high permeability. PNO has a strong interaction with phospholipids and acidic lipid molecules of the stratum corneum of skin, and thus can be enriched in gaps among keratinocytes and penetrate into the subepidermal layer or dermis layer through these gaps. After a conjugate or complex of PNO and an acidic group-containing biomolecule is applied to the skin, PNO, as a "locomotive", can be adsorbed on the surface of the skin and enriched, and then can penetrate through gaps among keratinocytes to reach the dermis layer, so as to allow the transdermal administration of small molecules, macromolecules such as hyaluronic acid and heparin with a molecular weight of 2,000,000, and even hyaluronic acid nanogels of more than 100 nm. As a result, the present disclosure completely breaks through the bottleneck that the existing conventional application of biomaterials such as hyaluronic acid can only be achieved through subcutaneous injections. The complex can dissociate subcutaneously to leave biomaterials such as hyaluronic acid in the dermis layer to maintain a shaping effect. Under an action of PNO, a conjugate produced by bonding PNO to a biomaterial molecule such as hyaluronic acid and heparin can also enter a circulation system after penetrating through the stratum corneum and dermis layer to achieve the systemic drug delivery.

Preferably, an acidic group is selected from one or more of a carboxylate group, a sulfonate group, a sulfate group, and a phosphate group.

Preferably, the acidic group-containing biomaterial includes natural and semi-natural polymers and a synthetic polymer; the natural and semi-natural polymers include hyaluronic acid, heparin, heparitin, chondroitin sulfate, sodium alginate, polysaccharide sulfate, carboxylated polysaccharide, and sulfated polysaccharide; and the synthetic polymer includes polyglutamic acid (PGA), polyaspartic acid (PAA), and an aspartic acid-glutamic acid copolymer.

Preferably, the acidic group-containing biomaterial has a molecular weight of 200 Da to 2,000 kDa. Further, the hyaluronic acid has a molecular weight of 500 Da to 1,000 kDa.

Preferably, the acidic group-containing biomaterial nanogel is a gel produced through chemical crosslinking or physical crosslinking of one or more acidic group-containing biomaterials.

Preferably, carboxyl, hydroxyl, and amino in the acidic group-containing biomaterial or the acidic group-containing biomaterial nanogel are bonded to the tertiary amine oxide group-containing polymer through chemical bonds, and the chemical bonds include an amide bond, an ester bond, an ether bond, a urea group, a thiourea group, and a carbamate group.

Preferably, a tertiary amine oxide group in the tertiary amine oxide group-containing polymer is an oxide of a saturated or unsaturated tertiary amine group, and the tertiary amine group is selected from one of N,N-dimethylamino, N,N-diethylamino, N,N-dipropylamino, *N,N-*methylethylamino, N-pyrrolidinyl, N-piperidinyl, N-morpholinyl, and pyridyl.

Preferably, the tertiary amine oxide group-containing polymer is a poly(meth)acrylate, a poly(meth)acrylamide, a polyamino acid, a polyester, or a polyethyleneimine. The PNO can be prepared as follows: preparing a tertiary amine group-containing polymer by a preparation method common in the polymer field, and then oxidizing the tertiary amine group into an N-oxide with an oxidant such as hydrogen peroxide or peracetic acid; or preparing a tertiary amine oxide group-containing monomer, and preparing the polymer by a conventional polymerization method.

Preferably, the tertiary amine oxide group-containing polymer is a homopolymer or copolymer with a linear or branched structure.

Preferably, the tertiary amine oxide group-containing polymer has a molecular weight of 300 Da to 50 kDa.

Preferably, a mass ratio of the acidic group-containing biomaterial or the acidic group-containing biomaterial nanogel to the tertiary amine oxide group-containing polymer is 1:10 to 10:1.

The present disclosure also provides a use of the efficient transdermal delivery system for an acidic group-containing biomaterial in preparation of a skin-permeable material for a cosmetic medical product.

The present disclosure also provides a use of the efficient transdermal delivery system for an acidic group-containing biomaterial in preparation of a skin-permeable medical auxiliary for skin care, repair, and drug delivery.

The present disclosure has the following beneficial effects:
The present disclosure can efficiently penetrate through the skin and other epithelial tissues to enter the subcutaneous tissues, and can allow the transdermal drug delivery without a subcutaneous injection. Accordingly, the present disclosure can achieve the simple and convenient nondestructive transdermal delivery, thereby enabling superior and lasting cosmetic medical and drug delivery effects.

### BRIEF DESCRIPTION OF THE DRAWINGS

FIG. 1 shows the preparation and representative structure for PNO with a terminal group functionalized in Examples 1.1 to 1.16, including the synthesis of OPDMA-NH₂ and the routes of preparing OPDMA-NHS, OPDMA-MA, and OPDMA-SH from OPDMA-NH₂, where the conventional OPDMA with a terminal group not functionalized can be prepared through the polymerization with a protective amino-free initiator;
FIG. 2 shows the random copolymerization of 2-(N,N-diethylamino)ethyl methacrylate (ODMA) and 2-(Boc amino)ethyl methacrylate to prepare OPDMA with amino or maleimido in a side chain in Example 1.21;
FIG. 3 shows the representative tertiary amine oxide group-containing polyamino acids prepared in Examples 1.25 to 1.28 (e: ester, and a: amide);
FIG. 4 shows the examples of dendritic macromolecules functionalized with a tertiary amine oxide group on a surface in Example 1.29;
FIG. 5 shows the routes of modifying hyaluronic acid and nanogels thereof with PNO;
FIG. 6 shows the particle size distribution measured by dynamic light scattering and electron microscopy image (A, B) of the nanogel prepared in Example 2.5 and the particle size distribution measured by dynamic light scattering and electron microscopy image (C, D) of the OPDMA (molecular weight: 5,000 Da)-modified nanogel prepared in Example 2.7;
FIG. 7 shows the stability of a hyaluronic acid/OPDMA complex at different pH values, where the complex remains stable at a pH of 2 to 4 and is dissociated at a high pH;

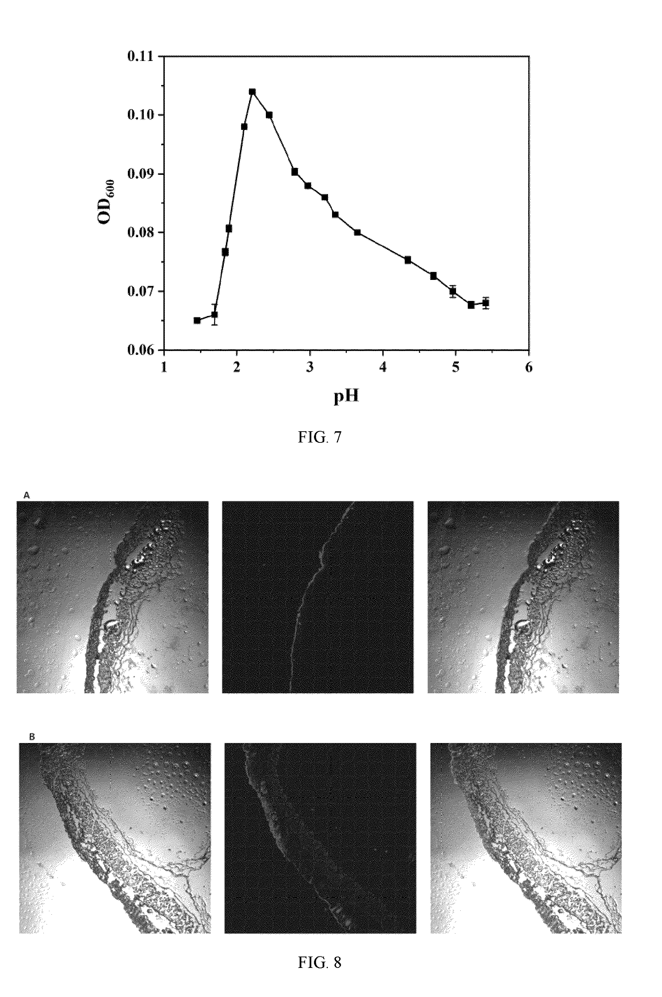

FIG. 8 shows the laser scanning confocal microscopy images of skin sections prepared from skins applied with a hyaluronic acid nanogel and an OPDMA-bonded hyaluronic acid nanogel for 4 h in Example 4.1, where (A) shows the sections of a skin treated with the nanogel prepared in Example 2.5 labeled by Cy5 for 4 h and (B) shows the sections of a skin treated with the OPDMA (molecular weight: 5,000 Da)-bonded nanogel prepared in Example 2.8 labeled by Cy5 for 4 h; the left shows the brightfield image, the middle shows the Cy5 fluorescence channel image, and the right shows the superposed image of the former two; and it can be seen that the conjugate can rapidly permeate to reach a subcutaneous tissue, while the hyaluronic acid itself does not have the ability to penetrate through a skin;

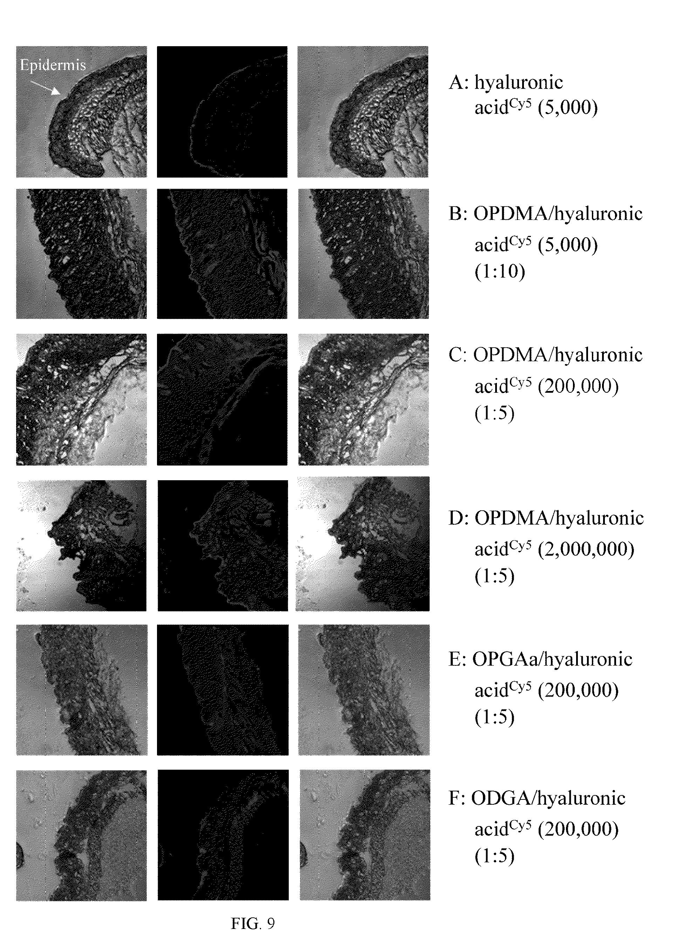

FIG. 9 shows the laser scanning confocal microscopy images of skin sections prepared from skins applied by a complex of a Cy5-labeled hyaluronic acid (A: molecular weight: 5,000 Da) or a Cy5-labeled hyaluronic acid with OPDMA (Examples 3.1 (B), 3.3 (C), and 3.4 (D)) or other polymers (Example 3.16 (E) and 3.17 (F)) in Example 4.1 for 4 h, where the left shows the brightfield image, the middle shows the Cy5 fluorescence channel image, and the right shows the superposed image of the former two; and it can be seen that the Cy5-labeled hyaluronic acid itself cannot penetrate through the skin, and after the hyaluronic acid is compounded with OPDMA or other PNOs, a large amount of Cy5-labeled hyaluronic acid enters the dermis layer;
FIG. 10 shows the hyaluronic acid content increases in skins of mice measured at different time points after hyaluronic acid/OPDMA (5:1; hyaluronic acid concentration: 0.5 mg/mL) is applied to the skins of mice;
FIG. 11 shows the daily skin changes recorded by images when 200 µL of a hyaluronic acid/OPDMA complex nanogel suspension (hyaluronic acid concentration: 0.5 mg/mL) is applied to dorsal skins of BALB/c mice twice a day;
FIG. 12 shows the skin statuses and skin morphologies of rhino mice applied with a hyaluronic acid/OPDMA complex nanogel suspension (hyaluronic acid concentration: 0.5 mg/mL), where 400 µL of a hyaluronic acid aqueous solution or a complex nanogel suspension (hyaluronic acid concentration: 0.5 mg/mL) is fixed on a rough dorsal skin of a mouse with a biological dressing to allow wet-dressing overnight (10 h) every day, and a skin change at a wet-dressed site is recorded;

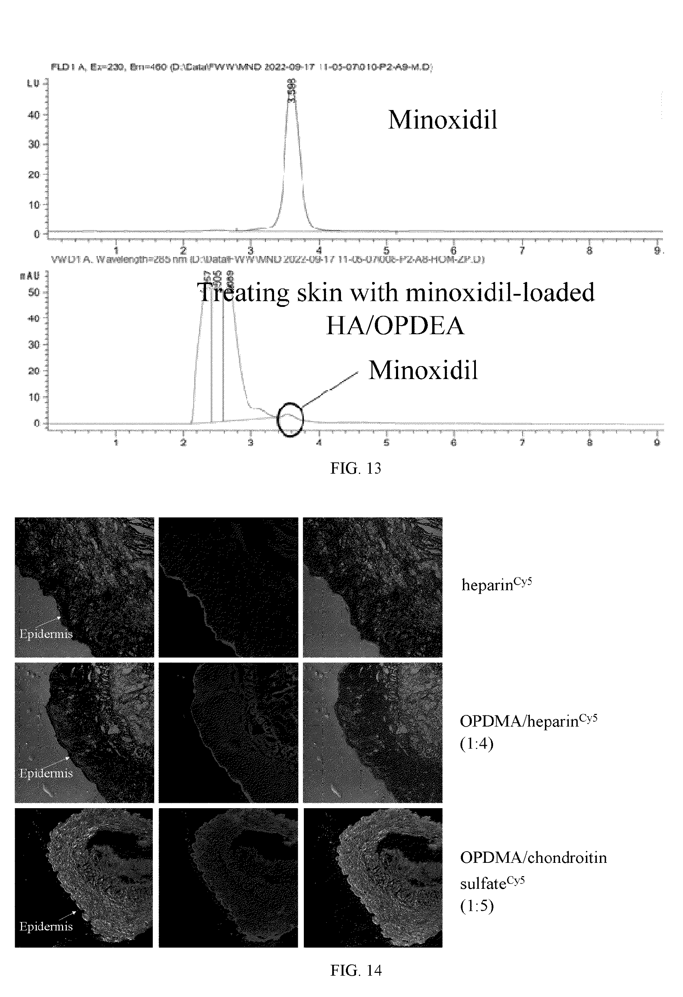

FIG. 13 shows the transdermal delivery of minoxidil by a minoxidil-loaded hyaluronic acid/OPDMA complex nanogel, where the complex nanogel is constructed with hyaluronic acid, OPDEA, and minoxidil in a mass ratio of 5:1:2; after a dorsal skin of a mouse is wet-dressed for 4 h, a skin tissue is collected and a dermis layer is separated; a tissue is homogenized and then tested by high-performance liquid chromatography (HPLC) for a content of minoxidil; minoxidil can be detected in a corresponding dermis layer; and no minoxidil is detected in a dermis layer of a skin coated with a minoxidil solution;
FIG. 14 shows the brightfield images (left), fluorescence microscopy images (middle), and superposed images of the former two (right) of mouse skins treated by a complex nanogel produced from OPDMA with Cy5-labeled heparin or chondroitin sulfate for 2 h, where heparin or chondroitin sulfate is largely enriched in a subcutaneous layer;
FIG. 15 shows the mechanism of the generation of a complex from PNO and an acidic group-containing macromolecule; and
FIG. 16 shows a superposed image of a fluorescence microscopy image and a brightfield image of a mouse skin surface applied with hyaluronic acid ^{Cy5}/OPDEA for 2 h, where it can be seen that hyaluronic acid^{Cy5}/OPDEA is abundantly enriched in gaps among keratinocytes.

### DETAILED DESCRIPTION OF THE EMBODIMENTS

The technical solutions of the present disclosure will be described in further detail below with reference to specific embodiments.

In the present disclosure, unless otherwise specified, all raw materials and devices adopted are commercially available or are commonly used in the art. All methods in the following embodiments are the conventional methods in the art, unless otherwise specified. It should be specifically noted that this example illustrates the preparation of functional group-containing PNO and the use of the functional group-containing PNO in synthesis of a conjugate or the use of labeled PNO in experiments. A complex is also prepared from functional group-free PNO. A corresponding preparation method is the same as above, except that an initiator without a functional group is adopted and the deprotection step is omitted.

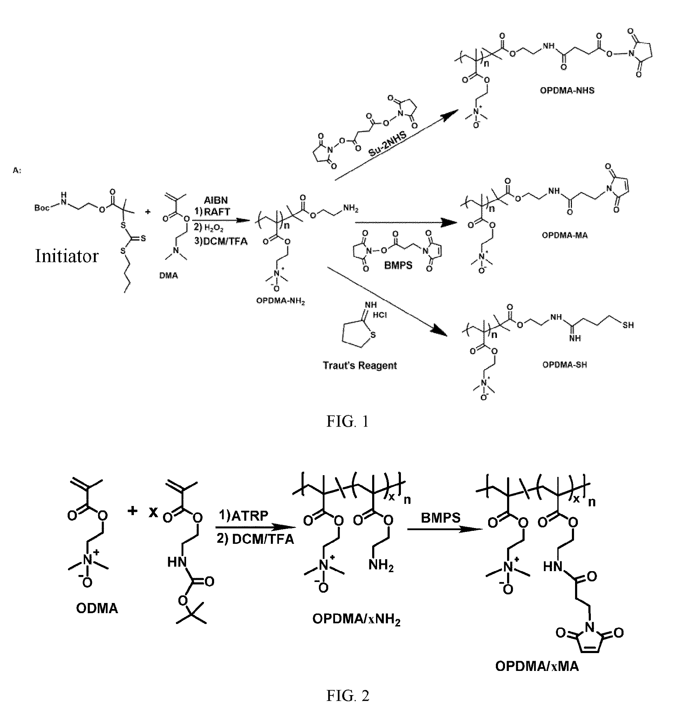

### I. Preparation of a tertiary amine oxide group-containing polymer (FIG. 1)

### 1. Preparation of a polymer including a reactive group and a tertiary amine oxide group

### Example 1.1 Poly[2-(N-oxide-N,N-dimethylamino)ethyl methacrylate] (OPDMA-NH₂) with primary amino as a terminal group was taken as an example for illustration:

Preparation of polymethacrylate-2-(N,N-dimethylamino)ethyl ester (PDMA-N-Boc) including terminal protective amino: 125 mg of [2-(N-tert-butoxycarbonylamino)]ethyl-2'-(butyltrithiocarbonate)-2'-methacrylate as a reversible addition-fragmentation chain transfer (RAFT) agent, 4.1 mg of azodiisobutyronitrile (AIBN), and 2.35 g of methacrylate-2-(N,N-dimethylamino)ethyl ester (DMA) were weighed and added to a 100 mL round-bottomed flask, and 20 mL of an anhydrous tetrahydrofuran solution was added. The reaction was allowed at 65°C for 12 h and then terminated. Concentration was conducted, and then precipitation was conducted with ice-cold n-hexane. A resulting precipitate was collected and vacuum-dried to produce PDMA-N-Boc. The molecular weight of the polymer was measured by gel permeation chromatography (GPC) to be 5,100 Da. Polymers with molecular weights from 1,000 Da to 50,000 Da could be prepared through polymerization with different monomers under similar conditions. Details were shown in the following table for Examples 1.2 to 1.7.

| Example | DMA (mmoL ) | RAFT (mmoL) | AIBN (mmoL) | Molecular weight of the polymer PDMA-*N*-Boc (Da) |
|---|---|---|---|---|
| Example 1.2 | 30 | 2.5 | 0.25 | 1100 |
| Example 1.3 | 30 | 1.25 | 0.125 | 1950 |
| Example 1.4 | 30 | 1 | 0.1 | 2100 |
| Example 1.5 | 30 | 0.25 | 0.025 | 9800 |
| Example 1.6 | 30 | 0.125 | 0.0125 | 19700 |
| Example 1.7 | 30 | 0.05 | 0.005 | 49900 |

1 g of the polymer PDMA-N-Boc prepared above was taken and added to a 25 mL flask, and 10 mL of 30% hydrogen peroxide was added. Stirring was conducted at room temperature for 3 h, and then dialysis was conducted. The dialysate was lyophilized to produce a polymer OPDMA-N-Boc in which the amino groups of PDMA-N-Boc was oxidized.

The OPDMA-N-Boc prepared above was dissolved in 5 mL of dichloromethane/5 mL of trifluoroacetic acid. The reaction was allowed at room temperature for 2 h. Precipitation was conducted with diethyl ether to produce a polymer OPDMA-NH₂ with an active amino group as a terminal group.

### 2. OPDMAs with other reactive terminal groups were prepared with OPDMA-NH₂

Example 1.8. 1 g of OPDMA-NH₂ was weighed and dissolved in 1 mL of PBS at pH 7.4, and 100 mg of a solution of N-β-maleimidopropyl-oxysuccinimide ester (BMPS) and triethylamine in DMSO was added. A reaction was allowed for 2 h at room temperature under stirring. A resulting reaction mixture was then purified with an ultrafiltration tube to produce an OPDMA polymer with terminal maleimido, namely, OPDMA-MA.

Similarly, OPDMA-NH₂ could react with molecules containing sulfhydryl, an isothiocyanate group, an epoxy group, a divinyl sulfone (DVS) group, and an activated ester group to prepare OPDMA with the isothiocyanate group, sulfhydryl, epoxy group, vinyl sulfone group, or activated ester group as a terminal group. Details were shown in the following table for Examples 1.9 to 1.13.

| Example | Reactive molecule example | Terminal group of the polymer | Polymer |
|---|---|---|---|
| Example 1.9 | p-phenylene diisothiocyanate | Isothiocyanate group | OPDMA-ITC |
| Example 1.10 | 2-Iminothiolane hydrochloride | Sulfhydryl | OPDMA-SH |
| Example 1.11 | Epichlorohydrin or 1,4-butanediol diglycidyl ether (BDDE) | Epoxy group | OPDMA-EP |
| Example 1.12 | DVS | Vinyl sulfone group | OPDMA-VS |
| Example 1.13 | Disuccinimidyl succinate | NHS-activated ester group | OPDMA-NHS |

3. Preparation of other tertiary amine oxide group-containing polymers: Polymers including tertiary amine oxide groups with other substituents could be prepared through the same steps as above. Details were shown in the following table for Examples 1.14 to 1.16., including poly[2-(N-oxide-N,N-dimethylamino)ethyl methacrylamide] (OPDMAA), poly[4-vinyl(N-oxide)pyridine] (OPVP), poly[2-(N-oxide-N-pyrrolidinyl)ethyl methacrylate] (OPPyA), etc.

| Example | Monomer | Polymer |
|---|---|---|
| Example 1.14 | 2-(*N,N*-diethylamino)ethyl methacrylamide | OPDEA |
| Example 1.15 | 4-Vinyl(N-oxide)pyridine | OPVP |
| Example 1.16 | Methacrylate-2-(*N*-pyrrolidinyl)ethyl ester | OPPyA |

A tertiary amine oxide group-containing monomer could be directly polymerized with the initiator system in Example 1.1 and then subjected to deprotection to directly prepare other tertiary amine oxide group-containing polymers with terminal amino. Details were shown in the following table for Examples 1.17 to 1.19.

| Example | Tertiary amine oxide group-containing monomer | Polymer |
|---|---|---|
| Example 1.17 | 2-(*N*-oxide-*N,N*-diethylamino)ethyl methacrylate | OPDEA-NH₂ |
| Example 1.18 | 4-Vinylpyridine *N*-oxide | OPVP-NH₂ |
| Example 1.19 | 1-Vinyl-1*H*-imidazole 3-oxide | Poly[1-vinyl-1*H*-imidazole 3-oxide] (OPVI-NH₂) |

Example 1.20. The polymerization of DMA (20 mmol) and 2-hydroxyethyl methacrylate (HEMA, 10 mmol) was initiated according to the initiator and conditions in Example 1.1 to prepare the corresponding random polymer of DMA and HEMA, namely, poly[DMA-co-HEMA]. A molecular weight of the random polymer was measured by GPC to be 5,500 Da. The polymer was oxidized and then deprotected according to the conditions and methods in Example 1 to prepare a tertiary amine oxide group-containing copolymer with terminal amino, namely, poly[ODMA-co-HEMA]-NH₂.

### Example 1.21. Preparation of a polymer with a reactive group on a side chain (FIG. 2)

With the OPDMA polymer as an example: ODMA (2.5 g), 2-(Boc amino)ethyl methacrylate (142 mg), cuprous bromide (17.66 mg), N,N,N',N",N"-pentamethyldiethylenetriamine (21.52 mg), and ethyl 2-bromo-2-methylpropionate (24.26 mg) (molar ratio: 100:5:1:1:1) were dissolved in 4 mL of methanol, and the reaction was allowed at 30°C for 3 h. Then dialysis was conducted with deionized water until a dialysate did not include copper ions. Lyophilization was conducted to produce a copolymer with a molecular weight of 12.5 kDa.

The polymer was dissolved in a mixed solution of 30 mL of chloroform/15 mL of trifluoroacetic acid, and the reaction was allowed for 4 h with stirring. The trifluoroacetic acid and the chloroform were removed through rotary evaporation. Washing was conducted with acetone three times, and then vacuum-drying was allowed to produce OPDEA with 5% (mol) of NH₂ on a side chain (OPDMA/5% NH₂).

OPDMA/5% NH₂ was dissolved in a small amount of N,N-dimethylformamide (DMF), and 200 µL of triethylamine and 50 mg of BMPS were added. A reaction was allowed overnight under stirring. Dialysis was conducted with a solution of DMF and water in 1:1 and deionized water, and lyophilization was conducted to produce OPDMA with 5% (mol) of maleimido on a side chain (OPDMA/5% MA).

The amount of the 2-(Boc amino)ethyl methacrylate (x) could be controlled to adjust a content of NH₂ or MA in the side chain.

According to the method in the literature (Athanasiou V et al., Synthesis and characterization of the novel Nε -9-fluorenylmethoxycarbonyl-1-lysine n-carboxy anhydride: Synthesis of well-defined linear and branched polypeptides. Polymers (Basel). 2020, 12: 2819), the ring-opening polymerization of N-carboxyanhydrides (NCAs) of amino acids was initiated with a protective amino-containing initiator, and the carboxyl or amino protective group on a side chain was removed to produce the corresponding polyamino acids with a terminal group functionalized. Details were shown in the following table for Examples 1.21 to 1.24.

| Example | Amino acid for preparing NCA | Polyamino acid | Molecular weight (Da) | Example | Bonded tertiary amine oxide group-containing molecule | Polyamino acid functionalized with a tertiary amine oxide |
|---|---|---|---|---|---|---|
| Example 1.21 | Glutamic acid | PGA | 9800 | 1.25 | 2-(*N*-oxide-*N,N-*dimethyl)ethanol or 2-(*N*-oxide-*N,N-*dimethyl)ethylam me | OPGA |
| Example 1.22 | Aspartic acid | PAA | 9200 | 1.26 | | OPAA |
| Example 1.23 | Lysine | Poly-L-lysine (PLL) | 9300 | 1.27 | | OPLL |
| Example 1.24 | Serine | Polyserine (PS) | 9700 | 1.28 | | OPS |

Example 1.25. PGA and 2-(N-oxide-N,N-dimethyl)ethanol (or amine) were subjected to an esterification or amidation reaction under the catalysis of a condensing agent [1-ethyl-(3-dimethylaminopropyl)carbodiimide hydrochloride] to produce poly[glutamic acid 2-(*N*-oxide-*N,N-*dimethyl)ethyl ester (OPGAe) or amide (OPGAa)] with a tertiary amine oxide group on a side chain.

Example 1.26. PAA and 2-(*N*-oxide-N,N-dimethyl)ethanol (or amine) were subjected to an esterification or amidation reaction under the catalysis of a condensing agent to produce poly[aspartic acid 2-(*N*-oxide-*N,N*-dimethyl)ethyl ester (or amide)] with a tertiary amine oxide group (OPAA).

Example 1.27. PLL was allowed to react with an excess amount of 1,1'-carbonyldiimidazole (1:5) overnight. The unreacted 1,1'-carbonyldiimidazole and by-products were removed. Then 2-(N-oxide-N,N-dimethyl)ethanol (or amine) (1:3) was added to allow a reaction for 48 h. Purification was then conducted to produce poly{*N*^{ε}-[2-(*N*-oxide-*N,N-*dimethyl)ethoxycarbonyl lysine} or poly{*N*^{ε}-[2-(*N*-oxide-*N,N*-dimethyl)ethylaminocarbonyl lysine} with a tertiary amine oxide group on a side chain (OPLL).

Example 1.28. Under the similar conditions in Example 27, PS was allowed to react with 1,1'-carbonyldiimidazole and then react with 2-(*N*-oxide-*N,N*-dimethyl)ethanol (or amine) to prepare the corresponding poly{O-[2-(*N*-oxide-*N,N*-dimethyl)ethoxycarbonyl serine} or poly{O-[2-(*N*-oxide-*N*,*N*-dimethyl)ethylaminocarbonyl serine} with a tertiary amine oxide group on a side chain (OPS).

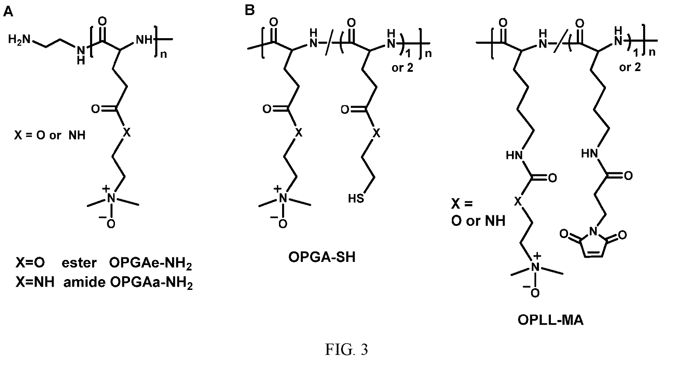

FIG. 3 shows the representative tertiary amine oxide group-containing polyamino acids prepared in Examples 1.25 to 1.28. With the reactions in Examples 1.9 to 1.13, sulfhydryl, maleimido, an isothiocyanate group, and an NHS-activated ester group could be introduced into a terminal group or a side chain of a polymer.

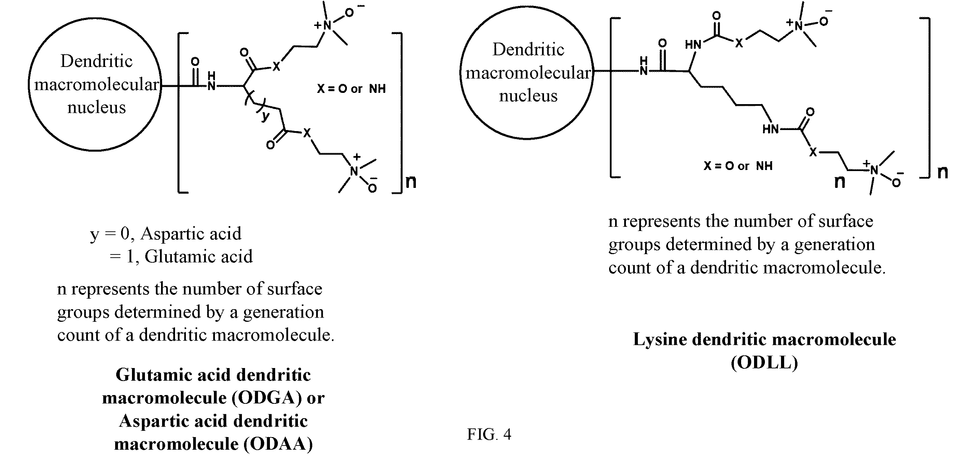

Example 1.29. Dendritic macromolecules functionalized with a tertiary amine oxide group on a surface (FIG. 4) Similarly, under the experimental conditions in Example 1.25, a dendritic macromolecule of PGA and 2-(N-oxide-N,N-dimethyl)ethanol (or amine) were subjected to an esterification or amidation reaction under the catalysis of a condensing agent to produce PGA dendritic macromolecule with 2-(N-oxide-N,N-dimethyl)ethyl ester or amide on a surface (ODGA). Under the reaction conditions in Example 1.26, a PAA dendritic macromolecule and 2-(N-oxide-N,N-dimethyl)ethanol (or amine) were subjected to an esterification or amidation reaction under the catalysis of a condensing agent to produce PAA dendritic macromolecule with 2-(N-oxide-N,N-dimethyl)ethyl ester (or amide) on a surface (ODAA). Under the reaction conditions in Example 1.27, a PLL dendritic macromolecule was allowed to react with an excess amount of 1,1'-carbonyldiimidazole (1:5) overnight. The unreacted 1,1'-carbonyldiimidazole and by-products were removed. Then 2-(N-oxide-N,N-dimethyl)ethanol (or amine) (1:3) was added to allow a reaction for 48 h. Purification was then conducted to produce a PLL dendritic macromolecule with 2-(*N*-oxide-*N,N*-dimethyl)ethoxycarbonyl or 2-(N-oxide-N,N-dimethyl)ethylaminocarbonyl on a surface (ODLL).

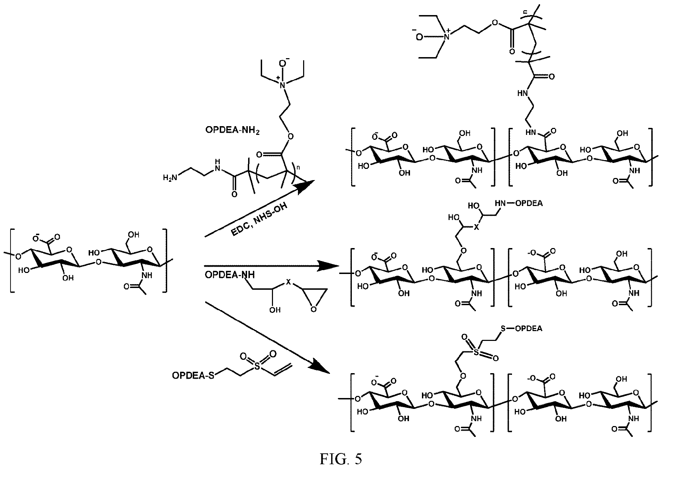

### II. Bonding of PNO to an acidic group-containing biomaterial to prepare a conjugate: Hyaluronic acid was taken as an example (FIG. 5).

Example 2.1 Preparation of OPDMA-modified hyaluronic acid by a carboxyl activation method in an aqueous solution: 1 g of hyaluronic acid, 10 mg of 1-ethyl-(-3-dimethylaminopropyl)carbodiimide (EDC), 10 mg of N-hydroxysuccinimide (NHS-OH), and 132 mg of OPDMA-NH₂ (molecular weight: 5,000 Da) were dissolved in 50 mL of deionized water, and stirring was conducted for 16 h at room temperature. A resulting system was heated to 50°C to allow a reaction for 1 h, and then cooled to room temperature. Washing was conducted with diethyl ether. Purification was conducted with a weakly-acidic cation exchange resin at a pH of 7.0. Then dialysis and lyophilization were conducted to produce hyaluronic acid with OPDMA (modification proportion: about 1% per hyaluronic acid unit) linked to carboxyl, namely, OPDMA-HA.

Example 2.2 Preparation of OPDMA-modified hyaluronic acid by a carboxyl activation method in an organic solvent: 3.79 g of hyaluronic acid, 25 mg of N,N'-dicyclohexylcarbodiimide, 15 mg of NHS-OH, and 15 mg of 4-dimethylaminopyridine (DMAP) were dissolved in 50 mL of anhydrous dimethyl sulfoxide (DMSO), and a reaction was allowed for 24 h at room temperature. 600 mg of OPDMA-NH₂ was added, and stirring was fully conducted, and a reaction was allowed at room temperature for 24 h. Dialysis and purification were conducted. Then lyophilization was conducted to produce OPDMA-modified hyaluronic acid with a modification proportion of about 1% per hyaluronic acid unit, namely, OPDMA-HA.

Example 2.3 Preparation of OPDMA-modified hyaluronic acid by a 1,4-butanediol diglycidyl ether (BDDE) method in an organic solvent: OPDMA-NH₂ (100 mg) was dissolved in 5 mL of ethanol, 10 mg of BDDE was added, and stirring was conducted for 5 h. Precipitation and washing were conducted multiple times with acetone. A product was added to 50 mL of a DMSO solution in which 3.79 g of hyaluronic acid and 200 mg of lithium carbonate were dissolved, and a reaction was allowed for 24 h. Dialysis and lyophilization were conducted to produce OPDMA-HA with an ether bond as a linking bond and an OPDMA modification proportion of 0.2% per hyaluronic acid unit.

Example 2.4 Preparation of OPDMA-modified hyaluronic acid by a DVS method: OPDMA-SH (100 mg) was dissolved in 5 mL of ethanol, 10 mg of DVS was added, and stirring was allowed for 1 h. Precipitation and washing were conducted multiple times with acetone. A product was dissolved in 5 mL of deionized water. A resulting solution was added to 50 mL of a 0.2 M NaOH solution in which 3.79 g of hyaluronic acid was dissolved, and a reaction was allowed for 1 h. Then dialysis and lyophilization were conducted to produce OPDMA-HA with thioether and ether bond formed by DVS as linking bonds and an OPDMA modification proportion of 0.2% per hyaluronic acid unit.

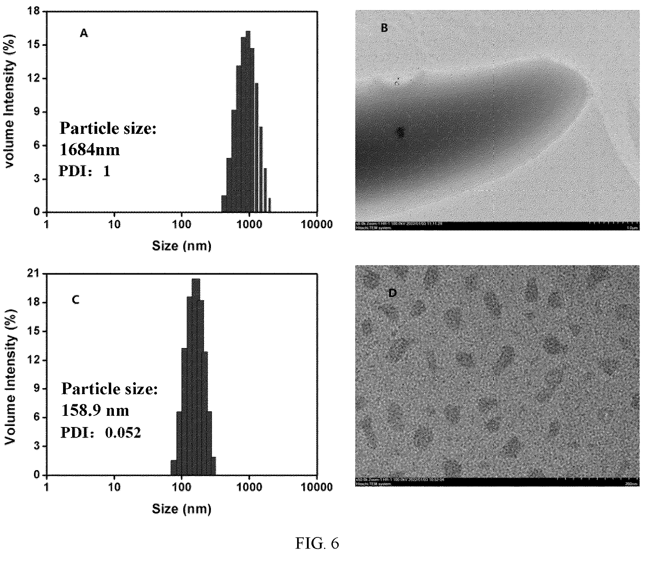

Example 2.5 Preparation of a BDDE-crosslinked hyaluronic acid gel: 1 g of hyaluronic acid was dissolved in 25 mL of a sodium hydroxide solution with a concentration of 0.2 M, 100 mg of BDDE was added, and stirring was conducted at room temperature for 16 h. A resulting system was heated to 50°C to allow a reaction for 1 h, and then cooled to room temperature. Washing was conducted with diethyl ether. Purification was conducted with a weakly-acidic cation exchange resin at a pH of 7.0 to produce the BDDE-crosslinked gel. As shown in FIG. 6, the gel formed was at a micron level.

Example 2.6 Preparation of a DVS-crosslinked hyaluronic acid gel: 1 g of hyaluronic acid was dissolved in 25 mL of a sodium hydroxide solution with a concentration of 0.2 M, 25 mg of DVS was added, and stirring was conducted at room temperature for 1 h. A weakly-acidic cation exchange resin was added, and a pH was adjusted to 7.0 to produce the DVS-crosslinked gel.

Example 2.7 OPDMA-modified crosslinked hyaluronic acid nanogel (method 1): The crosslinked hyaluronic acid in Example 2.5 was added to OPDMA-NH₂ (mass ratio: 5:1), heating was conducted to 50°C, and stirring was conducted for 3 h. OPDMA-NH₂ reacted with the unreacted epoxy group on the crosslinked hyaluronic acid to produce an OPDMA-modified hyaluronic acid nanogel. FIG. 6 shows that, unlike the original unmodified crosslinked hyaluronic acid, the modified nanogel has a relatively-uniform particle size changing from the micron level to about 150 nm, indicating that amino of OPDMA can effectively react with the residual epoxy group on the crosslinked hyaluronic acid to effectively disperse the nanogel.

Example 2.8 OPDMA-modified crosslinked hyaluronic acid nanogel (method 2): The crosslinked hyaluronic acid in Example 2.6 was added to OPDMA-NH₂ (or OPDMA-SH, mass ratio: 5:1), and stirring was conducted for 3 h. OPDMA-NH₂ (or SH) reacted with the unreacted DVS on the nanogel to produce the OPDMA-modified hyaluronic acid nanogel.

Example 2.9 OPDMA-modified crosslinked hyaluronic acid nanogel (method 3): 100 mg of OPDMA-NH₂ was dissolved in 5 mL of methanol. 10 mg of BDDE was added and stirring was conducted for 5 h, or 10 mg of DVS was added and stirring was conducted for 1 h. Precipitation and washing were conducted multiple times with acetone to produce OPDMA with a terminal epoxy or vinyl sulfoxide group. The OPDMA was added to a 0.2 M sodium hydroxide solution including 1 g of hyaluronic acid, and stirring was conducted for 1 h. 25 mg of BDDE or DVS was added, and stirring was further conducted at room temperature for 1 h. A weakly-acidic cation exchange resin was added, and a pH was adjusted to 7.0 to produce the OPDMA-modified hyaluronic acid nanogel.

**Table 1 Hyaluronic acid nanogels prepared under different conditions**

| Example | Polymer PNO, molecular weight (Da) | Hyaluronic acid (molecular weight/Da) | Particle size of a nanogel (nm) |
|---|---|---|---|
| 2.5 | - | 5,000 | 1680 |
| | - | 50,000 | 3210 |
| 2.6 | - | 5,000 | 1520 |
| | - | 50,000 | 2870 |
| | - | 500,000 | Gel |
| 2.7 | Poly[2-(N-oxide-N,N-dimethylamino)ethyl methacrylate] (OPDMA-NH₂), 5,000 | 5,000 | 159 |
| | OPDMA-NH₂, 5,000 | 50,000 | 220 |
| | OPDMA-NH₂, 10,000 | 50,000 | 255 |
| | OPDMA-NH₂, 25,000 | 50,000 | 220 |
| | Poly[2-(*N*-oxide-*N,N*-diethylamino)ethyl methacrylate] (OPDEA-NH₂), 5,000 | 5,000 | 168 |
| | Poly[2-*N*-(*N*-oxide-pyrrolidinyl)ethyl methacrylate](OPPyA-NH₂), 10,000 | 5,000 | 145 |
| | OPVP-NH₂, 7,000 | 5,000 | 133 |
| | OPGA-NH₂, 10,000 | 5,000 | 142 |
| 2.8 | OPDMA-NH₂, 5,000 | 5,000 | 122 |
| | OPDMA-NH₂, 10,000 | 50,000 | 215 |
| | OPDMA-SH, 5,000 | 5,000 | 128 |
| 2.9 | OPDMA-NH₂ + BDDE, 5,000 | 50,000 | 148 |
| | OPDMA-NH₂ + DVS, 5,000 | 50,000 | 130 |

Example 2.10: Fluorescent labeling of hyaluronic acid or a hyaluronic acid nanogel EDC and NHS-OH were added to cross-linked hyaluronic acid or a nanogel thereof, and stirring was conducted at room temperature for 8 h. A Cy5 fluorescent dye including an amino group (Cy5-NH₂) was added (mass ratio: 1%), and stirring was conducted at room temperature in the dark for 24 h. Water was added repeatedly for dilution. Ultrafiltration centrifugation was conducted to remove the unreacted EDC, NHS-OH, and Cy5 until a filtrate was colorless.

### III. Preparation of complexes of PNOs with acidic group-containing biomaterials

Example 3.1 Preparation of a complex of OPDMA with hyaluronic acid or a hyaluronic acid nanogel: A 1 mg/mL OPDMA aqueous solution and aqueous solutions of hyaluronic acid samples with different molecular weights were prepared and mixed according to the corresponding OPDMA/hyaluronic acid mass ratio to produce the respective complexes. A particle size of a complex was determined by a NanoZS particle size analyzer.

**Table 2 Complexes of PNOs with acidic group-containing biomacromolecules such as hyaluronic acid**

| Example | PNO, molecular weight (Da) | Acidic group-containing macromolecule and molecular weight thereof (Da) | Mass ratio | Particle size of a complex (nm) |
|---|---|---|---|---|
| 3.1 | OPDMA, 5,000 | Hyaluronic acid, 5,000 | 1:10 | 171 |
| 3.2 | OPDMA, 5,000 | Hyaluronic acid, 5,000 | 1:5 | 158 |
| 3.3 | OPDMA, 5,000 | Hyaluronic acid, 200,000 | 1:5 | 208 |
| 3.4 | OPDMA, 5,000 | Hyaluronic acid, 2,000,000 | 1:5 | About 1,000, non- |
| 3.5 | OPDMA, 1,000 | Hyaluronic acid, 200,000 | 1:5 | 184 |
| 3.6 | OPDMA, 25,000 | Hyaluronic acid, 200,000 | 1:5 | 162 |
| 3.7 | OPDMA, 5,000 | Hyaluronic acid, 200,000 | 3:1 | 178 |
| 3.8 | OPDMA, 5,000 | Hyaluronic acid, 200,000 | 10:1 | Non-uniform |
| 3.9 | OPDMA, 5,000 | Heparin, 5,000 | 1:4 | 86 |
| 3.10 | OPDMA, 5,000 | Heparin, 15,000 | 1:4 | 125 |
| 3.11 | OPDMA, 5,000 | Sodium alginate, about 50,000 | 1:5 | 156 |
| 3.12 | OPDMA, 5,000 | Chondroitin sulfate, about 100,000 | 1:5 | 183 |
| 3.13 | OPDMA, 5,000 | PGA, 50,000 | 1:5 | 401 |
| 3.14 | OPPyA, 5,000 | Hyaluronic acid, 200,000 | 1:5 | 145 |
| 3.15 | OPVP, 5,000 | Hyaluronic acid, 200,000 | 1:5 | 138 |
| 3.16 | OPGA, 15,000 | Hyaluronic acid, 200,000 | 1:5 | 144 |
| 3.17 | ODGA (generation 5) | Hyaluronic acid, 200,000 | 1:5 | 167 |
| 3.18 | ODLL (generation 5) | Hyaluronic acid, 200,000 | 1:5 | 155 |
| 3.19 | OPDMA, 5,000 | Hyaluronic acid nanogel | 1:3 | 155 |

### Example 3.2 Evaluation of pH-stability of an OPDMA/hyaluronic acid complex

A 1 mg/mL /hyaluronic acid (1:5) complex aqueous solution was prepared. A pH of the solution was accurately adjusted in a range of 1.45 to 5.4, and the light absorption (OD₆₀₀) of the solution at 600 nm under each pH value was measured, so as to evaluate the pH-stability of the OPDMA/hyaluronic acid complex. As shown in FIG. 7, the OPDMA/hyaluronic acid complex remained stable at a pH of 2 to 4, and was dissociated at a pH higher than 4 or lower than 2.

### IV. Evaluation of transdermal abilities of conjugates and complexes of PNOs with acidic group-containing biomaterials

### Transdermal evaluation of fluorescently-labeled crosslinked hyaluronic acid nanogels

BALB/c mice were selected and subjected to dehairing. A dehaired region on a mouse was coated with a solution of a conjugate or complex of PNO. 4 h later, the mice were sacrificed, and a skin tissue was collected, fixed with 4% paraformaldehyde, and then frozen-sectioned. Skin sections were subjected to fluorescence analysis by a laser scanning confocal microscope.

As shown in FIG. 8, hyaluronic acid and hyaluronic acid gels of any molecular weight could not penetrate through the skin and could only stay in the epidermal layer. However, hyaluronic acid with a low molecular weight, hyaluronic acid with a molecular weight of 2000,000 Da, and even nanogels that each were bonded with OPDMA could penetrate through the skin and reach the dermis layer. Similarly, hyaluronic acid and nanogels that were modified with other N-oxide group-containing polymers could also effectively permeate the skin.

As shown in FIG. 9, complexes of hyaluronic acid with a molecular weight of 5,000 Da to 2,000,000 Da or nanogel particles thereof with OPDMA and other PNO-type polymers could also efficiently penetrate through the stratum corneum of skin and enter a dermal tissue after being applied to the skin.

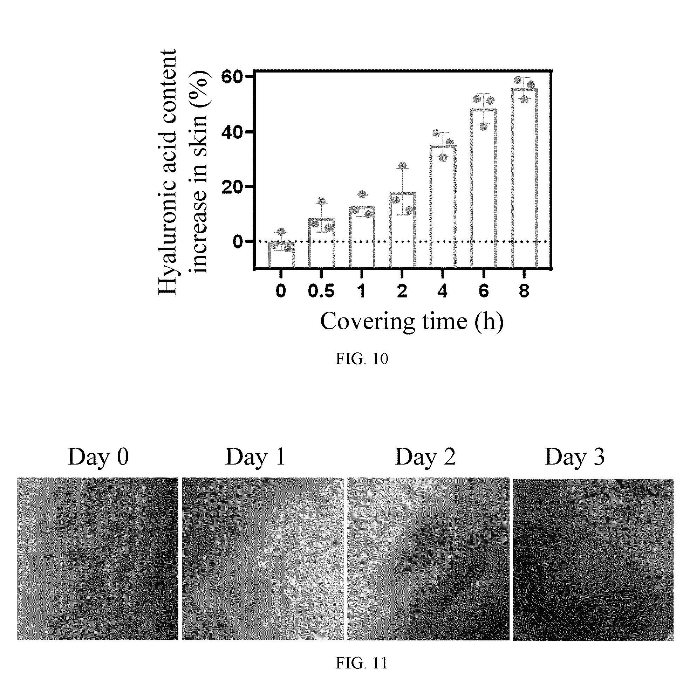

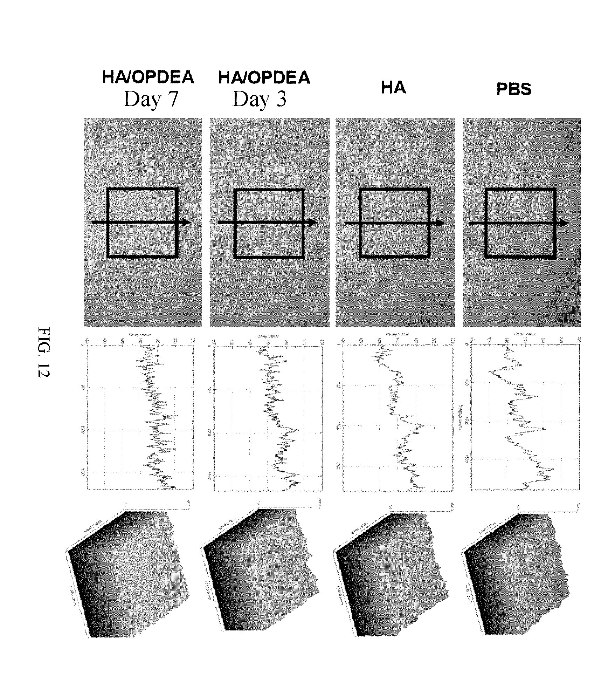

After the application to skin, a content of hyaluronic acid in the skin gradually increased over time, and could increase by 60% within 8 h (FIG. 10). 200 µL of a complex suspension (hyaluronic acid concentration: 0.5 mg/mL) was applied to a dorsal skin of a mouse twice a day. It could be observed that the skin was gradually plump (FIG. 11). A rhino mouse model with naturally-wrinkled skin was further adopted for an experiment. 400 µL of a hyaluronic acid aqueous solution or a hyaluronic acid/OPDMA (5:1) complex nanogel suspension (hyaluronic acid concentration: 0.5 mg/mL) was wet-dressed on the rough dorsal skin of a mouse overnight (10 h) every day, and a skin change at a wet-dressed site was recorded. A skin roughness was measured with ImageJ. It was found that the application of the hyaluronic acid/OPDMA (5:1) complex nanogel suspension could significantly reduce the skin roughness (FIG. 12).

A complex produced from hyaluronic acid and PNO could further carry a drug and deliver the drug transdermally to a subcutaneous layer. With the delivery of minoxidil as an example, a drug-containing complex nanogel was prepared with hyaluronic acid, OPDEA, and minoxidil in a mass ratio of 5:1:2. The drug-containing complex nanogel was wet-dressed on a back of a mouse for 4 h. Then a skin tissue was collected, and a dermis layer was separated. Homogenization was conducted, and a minoxidil content was analyzed by HPLC. It could be known that the dermis layer included minoxidil (FIG. 13). In contrast, no minoxidil was detected in the dermis of skin directly coated with a minoxidil solution. It indicated that the hyaluronic acid/OPDEA complex could carry minoxidil to penetrate through the skin for drug delivery.

Other acidic group-containing macromolecules or synthetic polymers, such as heparin, chondroitin sulfate, and PGA, were also combined with PNO to produce nanoparticles. These nanoparticles could also quickly penetrate through the stratum corneum and enter the dermis after being applied to skin, as shown in FIG. 14.

If the skin was first treated with an aqueous solution at a pH of about 3, a transdermal ability of each complex could be further improved.

### V. PNO-mediated transdermal mechanism of acidic group-containing biomaterials

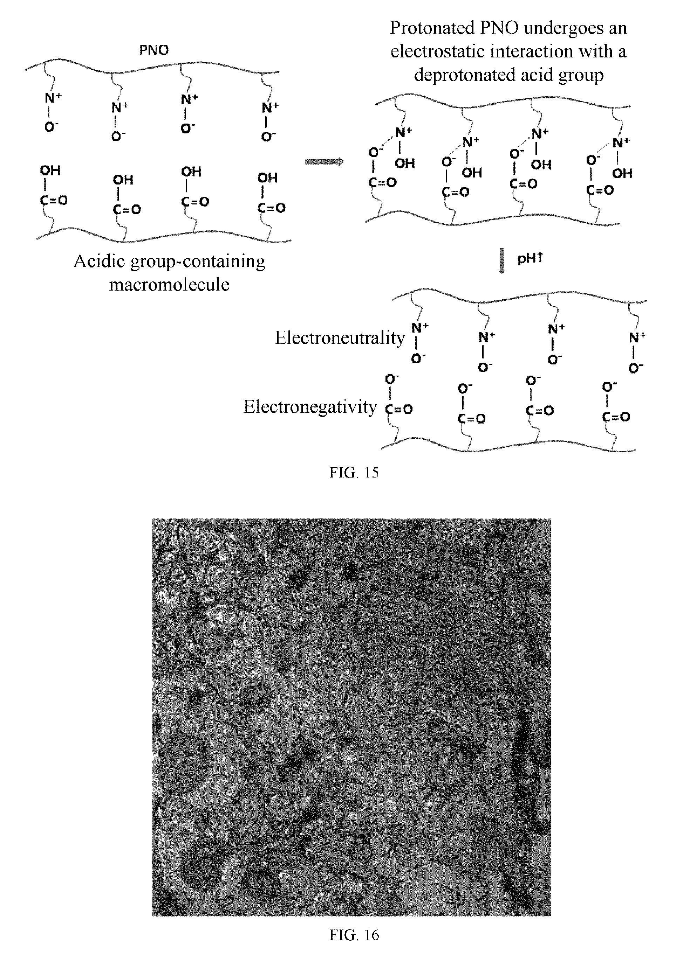

A ≡N⁺-O⁻ group of PNO is protonated into N⁺-OH by an acidic group in an acidic group-containing macromolecule. As a result, PNO can undergo an electrostatic interaction with a deprotonated negatively-charged acid group to produce a complex (FIG. 15). As a pH increases, the PNO becomes electrically neutral, and the complex is dissociated (FIG. 7). The skin surface is weakly acidic and has an average pH of about 5.8. Moreover, the stratum corneum of skin includes a large amount of keratin, a hydroxy fatty acid with 16 to 18 carbons. As a result, PNO is enriched on keratin based on the mechanism shown in FIG. 15, and thus an acidic group-containing macromolecule carried by PNO is enriched on keratin (FIG. 16). The complex enriched on keratin penetrates through the stratum corneum and permeates subcutaneously. After reaching the subepidermal layer or dermis layer with a pH close to physiological neutrality, the complex is dissociated, macromolecules such as hyaluronic acid remain in the subepidermal layer or dermis layer, and PNO further enters the blood and is excreted. However, a PNO-biomacromolecule conjugate can enter the blood and the systemic circulation due to an action of PNO.

### VI. Evaluation of enzyme-stability of polymer-modified hyaluronic acid and nanogels thereof

0.5 g of hyaluronic acid or polymer-modified hyaluronic acid or a complex was taken, hyaluronidase was added at 0.5 U/mL, and degradation was allowed at 37°C for 24 h. 0.2 mL of a degradation solution was taken, and 4.8 mL of absolute ethanol was added. High-speed centrifugation was conducted, and 1 mL of a resulting supernatant was collected and diluted with PBS to 5 mL as a solution A. 0.5 g of a hyaluronic acid gel was taken and added to 10 mL of a 0.5 mol/L sulfuric acid solution, and hydrolysis was allowed in a boiling water bath for 15 min. Cooling was allowed, and then dilution was conducted to 100 mL as a solution B. Mass concentrations of uronic acid in the above two solutions were determined by the modified carbazole method. Degradation rate = mass concentration of uronic acid in the solution A/mass concentration of uronic acid in the solution B. Experimental results showed that, within 24 h under the above conditions, 60% of the hyaluronic acid was degraded, but only 20% of the OPDMA-modified hyaluronic acid or the nano-complex was degraded, and the degradation was further slowed down with the increase of polymer chains. It indicates that the modification or compounding of the polymer can indeed promote the resistance of hyaluronic acid to enzymolysis.

The above examples are merely preferred solutions of the present disclosure and are not intended to limit the present disclosure in any form, and other variations and modifications may be made without departing from the technical solutions as set forth in the appended claims.

## Claims

1. An efficient transdermal delivery system for an acidic group-containing biomaterial, which is produced by bonding a tertiary amine oxide group-containing polymer to an acidic group-containing biomaterial or an acidic group-containing biomaterial nanogel, wherein the tertiary amine oxide group-containing polymer has a structural formula shown in any one of formulas I to III: 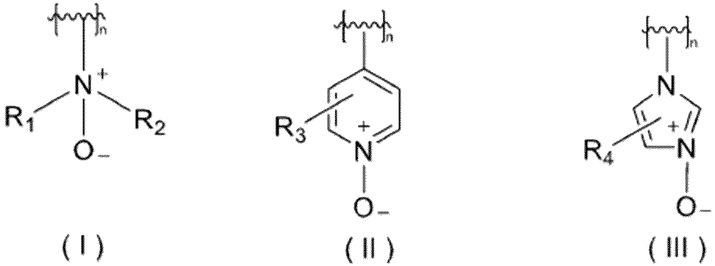 wherein
R₁ and R₂ each are selected from C₁-C₆ alkyl, substituted alkyl, aryl, or substituted aryl; and
R₃ and R₄ each are selected from hydrogen, halogen, cyano, alkoxy, C₁-C₆ alkyl, substituted alkyl, aryl, or substituted aryl.

2. An efficient transdermal delivery system for an acidic group-containing biomaterial, wherein the efficient transdermal delivery system is a complex produced by mixing the acidic group-containing biomaterial or an acidic group-containing biomaterial nanogel with a tertiary amine oxide group-containing polymer, wherein the tertiary amine oxide group-containing polymer has a structural formula shown in one of formulas I to III: 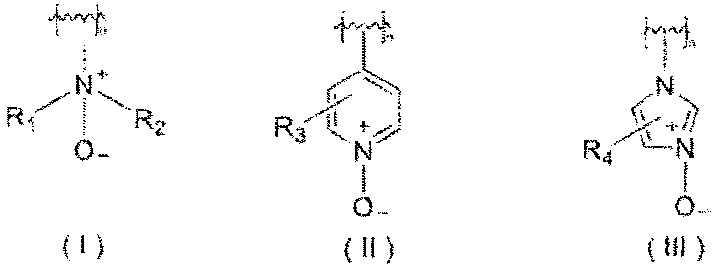 wherein
R₁ and R₂ each are selected from C₁-C₆ alkyl, substituted alkyl, aryl, or substituted aryl; and
R₃ and R₄ each are selected from hydrogen, halogen, alkoxy, cyano, C₁-C₆ alkyl, substituted alkyl, aryl, or substituted aryl.

3. The efficient transdermal delivery system for the acidic group-containing biomaterial according to claim 1, wherein an acidic group is selected from one or more of a carboxylate group, a sulfonate group, a sulfate group, and a phosphate group.

4. The efficient transdermal delivery system for the acidic group-containing biomaterial according to claim 1, wherein the acidic group-containing biomaterial comprises natural or semi-natural polymers or a synthetic polymer; the natural and semi-natural polymers comprise hyaluronic acid, heparin, heparitin, chondroitin sulfate, sodium alginate, polysaccharide sulfate, carboxylated polysaccharide, and sulfated polysaccharide; and the synthetic polymer comprises polyglutamic acid (PGA), polyaspartic acid (PAA), and an aspartic acid-glutamic acid copolymer.

5. The efficient transdermal delivery system for the acidic group-containing biomaterial according to claim 3, wherein the acidic group-containing biomaterial has a molecular weight of 200 Da to 2,000 kDa.

6. The efficient transdermal delivery system for the acidic group-containing biomaterial according to claim 3, wherein the hyaluronic acid has a molecular weight of 500 Da to 1,000 kDa.

7. The efficient transdermal delivery system for the acidic group-containing biomaterial according to claim 1, wherein the acidic group-containing biomaterial nanogel is a gel produced through chemical crosslinking or physical crosslinking of one or more acidic group-containing biomaterials.

8. The efficient transdermal delivery system for the acidic group-containing biomaterial according to claim 1, wherein carboxyl, hydroxyl, or amino groups in the acidic group-containing biomaterial or the acidic group-containing biomaterial nanogel are bonded to the tertiary amine oxide group-containing polymer through chemical bonds, and the chemical bonds comprise an amide bond, an ester bond, an ether bond, a urea group, a thiourea group, or a carbamate group.

9. The efficient transdermal delivery system for the acidic group-containing biomaterial according to claim 1, wherein a tertiary amine oxide group in the tertiary amine oxide group-containing polymer is an oxide of a saturated or unsaturated tertiary amine group, and the saturated or unsaturated tertiary amine group is selected from one of N,N-dimethylamino, N,N-diethylamino, N,N-dipropylamino, N,N-methylethylamino, N-pyrrolidinyl, N-piperidinyl, N-morpholinyl, and pyridyl.

10. The efficient transdermal delivery system for the acidic group-containing biomaterial according to claim 1, wherein the tertiary amine oxide group-containing polymer is a poly(meth)acrylate, a poly(meth)acrylamide, a polyamino acid, a polyester, or a polyethyleneimine.

11. The efficient transdermal delivery system for the acidic group-containing biomaterial according to claim 1, wherein the tertiary amine oxide group-containing polymer is a homopolymer or copolymer with a linear or branched structure.

12. The efficient transdermal delivery system for the acidic group-containing biomaterial according to claim 1, wherein the tertiary amine oxide group-containing polymer has a molecular weight of 300 Da to 50 kDa.

13. The efficient transdermal delivery system for the acidic group-containing biomaterial according to claim 1, wherein a mass ratio of the acidic group-containing biomaterial or the acidic group-containing biomaterial nanogel to the tertiary amine oxide group-containing polymer is 1: 10 to 10:1.

14. A use of the efficient transdermal delivery system for the acidic group-containing biomaterial according to claim 1 or 2 in preparation of a skin-permeable material for a cosmetic medical product.

15. A use of the efficient transdermal delivery system for the acidic group-containing biomaterial according to claim 1 or 2 in preparation of a skin-permeable medical auxiliary for skin care, repair, and drug delivery.
